# EUROPEAN PATENT SPECIFICATION

(11) **EP 2 012 764 B1**
(45) Date of publication and mention of the grant of the patent: **12.01.2011**
(21) Application number: 07724562.9
(22) Date of filing: 25.04.2007
(51) Int. Cl.: A61K 31/137, A61P 29/00, A61K 31/167

(54) **PHARMACEUTICAL COMBINATION COMPRISING 3-(3-DIMETHYLAMINO-1-ETHYL-2-METHYL-PROPYL) -PHENOL AND PARACETAMOL**
PHARMAZEUTISCHE KOMBINATION MIT 3- (3-DIMETHYLAMINO-1-ETHYL-2-METHYL-PROPYL)-PHENOL UND PARACETAMOL
COMBINAISON PHARMACEUTIQUE COMPRENANT DU 3-(3-DIMÉTHYLAMINO-1-ÉTHYL-2-MÉTHYLPROPYL)-PHÉNOL ET DU PARACÉTAMOL

(30) Priority: 28.04.2006 EP 06008851
(43) Date of publication of application: 14.01.2009
(73) Proprietor: Grünenthal GmbH, 52078 Aachen (DE)
(72) Inventor: BLOMS-FUNKE, Petra, 52146 Würselen (DE); SCHIENE, Klaus, 41363 Jüchen (DE)
(74) Representative: Brosch, Oliver
(86) International application number: PCT/EP2007/003632
(87) International publication number: WO 2007/128413

(56) References cited:
- WO-A-2004/047823
- DE-A1- 4 426 245
- "THE MERCK MANUAL OF DIAGNOSIS AND THERAPY; 17TH Edition" 1999, MERCK RESEARCH LABORATORIES , NEW JERSEY, USA , XP002401271 pages1363-1365 Ch. 167 "PAIN"

## Description

The present invention relates to a combination comprising as components (a) the compound 3-(3-Dimethylamino-1-ethyl-2-methyl-propyl)-phenol, and (b) Paracetamol a pharmaceutical formulation and a dosage form comprising said combination as well as a method of treating pain, e.g. chronic or acute pain, **characterized in that** components (a) and (b) are administered simultaneously or sequentially to a mammal, wherein component (a) may be administered before or after component (b) and wherein components (a) or (b) are administered to the mammal either via the same or a different pathway of administration.

The treatment of chronic and acute pain conditions is extremely important In medicine. There is currently a worldwide demand for additional, not exclusively opioid-based, but highly effective pain treatment. The urgent need for action for patient-oriented and purposeful treatment of pain conditions, this being taken to mean the successful and satisfactory treatment of pain for the patient, is documented In the large number of scientific papers which have recently appeared in the field of applied analgesics and fundamental research work on nociception.

Even if the analgesics that are currently used for treating pain, for example opioids. NA- and 5HT-reuptake inhibitors, NSAIDS and COX inhibitors, are analgeslcally effective, side effects nevertheless sometimes occur. WO 2004/047823 describes substance combinations comprising certain analgesics including 1-phenyl-3-dimethylamino-propane compounds and COX-II Inhibitors, which show super-additive effects upon administration. Due to the super-additive effect the overall dose and accordingly the risk of undesired side effects can be reduced.

DE 44 26 245 A1 discloses 1-Phenyl-3-dimethylamino-propane compounds having pharmacological activity.

"The Merck Manual of Diagnosis and Therapy"; 17th edition, 1999, Merck Research Laboratories, New Jersey, USA describes the use of Paracetamol for the treatment of pain.

Thus, it was an object of the present Invention to find further combinations that are suitable for the treatment of pain and which preferably exhibit fewer undesired side effects compared to its individual components, if administered in effective doses.

It has been found that a combination comprising (a) the compound 3-(3-Dimethylamino-1-ethyl-2-methyl-propyl)-phenol, and (b) Paracetamol or a derivative thereof exhibits an analgesic effect. If these components are present in the composition in such a weight ratio that a synergistic effect is observed after administration to the patients, the overall administered dose may be lowered, so that fewer undesired side-effects will occur.

Accordingly, the present invention relates to a pharmaceutical combination comprising as components
(a) 3-(3-Dimethylamino-1-ethyl-2-methyl-propyl)-phenol of formula (I) 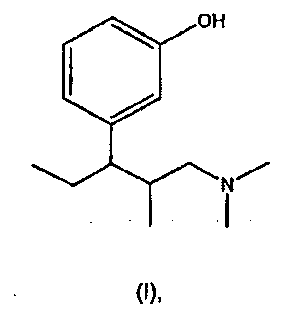 optionally in form of one of its pure stereolsomers, in particular an enantiomer or a diastereomer, a racemate or in form of a mixture of its stereoisomers, in particular enantiomers and/or diastereomers in any mixing ratio, or any corresponding acid addition salt thereof, or any solvate thereof, and
(b) Paracetamol .

In an embodiment of the inventive combination component (a) is selected from
(1R,2R)-3-(3-Dimethylamino-1-ethyl-2-methyl-propyl)-phenol,
(1S,2S)-3-(3-Dimethylamino-1-ethyl-2-methyl-propyl)-phenol,
(1R,2S)-3-(3-Dimethylamino-1-ethyl-2-memyl-propyl)-phenol,
(1S,2R)-3-(3-Dimethylamino-1-elhyl-2-methyl-propyl)-phenol, and any mixture thereof.

In another embodiment of the inventive combination component (a) is selected from
(1R,2R)-3-(3-Dimethylamino-1-ethyl-2-methyl-propyl)-phenol, and
(1S,2S)-3-(3-Dimethylamino-1-ethyl-2-methyl-propyl)-phenol, and any mixture thereof.

In yet another embodiment the inventive combination comprises
(a) the compound (1R,2R)-3-(3-Dimethylamino-1-ethyl-2-methyl-propyl)-phenol of
   formula (I'), 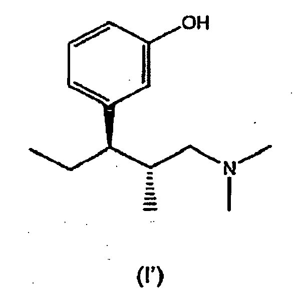 or an acid addition salt thereof, and
(b) Paracetamol.

The compound 3-(3-Dimethylamino-1-ethyl-2-methyl-propyl)-phenol of formula (I), its stereoisomers and corresponding salts thereof as well as methods for their preparation are well known, for example, from US 6,248,737 B1. The respective parts of the description are hereby incorporated by reference and form part of the present disclosure.

The definition of component (a) as used herein includes the compound 3-(3-Dimethylamino-1-ethyl-2-methyl-propyl)-phenol and its stereoisomers in any possible form, thereby particularly including solvates, acid addition salts and corresponding solvates and polymorphs thereof.

If component (a) is present as mixture of enantiomers, such a mixture may contain the enantiomers in racemic or non-racemic form. A non-racemic form could, for example, contain the enantiomers in a ratio of 60: 40, 70:30, 80:20 or 90:10.

The compound 3-(3-Dimethylamino-1-ethyl-2-methyl-propyl)-phenol and its stereoisomers according to component (a) may be present in the inventive pharmaceutical composition in form of an acid addition salt, whereby any suitable acid capable of forming such an addition salt may be used.

The conversion of the compound 3-(3-Dimethylamino-1-ethyl-2-methyl-propyl)-phenol into a corresponding addition salt via reaction with a suitable acid may be effected in a manner well known to those skilled in the art. Suitable acids include but are not limited to hydrochloric acid, hydrobromic acid, sulfuric acid, methanesulfonic acid, formic acid, acetic acid, oxalic acid, succinic acid, tartaric acid, mandelic acid, fumaric acid, lactic acid, citric acid, glutamic acid and/or aspartic acid. Salt formation is preferably effected in a solvent, for example diethyl ether, diisopropyl ether, alkyl acetates, acetone and/or 2-butanone. Moreover, trimethylchlorosilane in aqueous solution is also suitable for the preparation of hydrochlorides.

It is known to those skilled in the art that the analgesic action of non-steroidal anti-inflammatory drugs (NSAIDs) is due to the inhibition of the enzymatic production of prostaglandins, wherein Cyclooxygenase (COX) is the key enzyme in the conversion of arachidonic acid derived from lipids of the cell membrane to prostaglandins and other eicosanoids. COX exists in two different isoforms characterized by different expression patterns. COX-I is constitutively expressed in many cells of the body and responsible mainly for the production of eicosanoids serving normal physiological functions. COX-II expression is induced during inflammation and also COX-II is expressed in the central nervous system.

Paracetamol does not show any significant anti-inflammatory activity and is accordingly not considered to be NSAID.

The term paracetamol, also known as acetaminophen, as used herein includes this compound in any possible form, thereby including solvates and polymorphs thereof.

Paracetamol and its derivatives Propacetamol and Phenidine as well as processes for their preparation are well known in the art, for example from E. Friderichs et al. "Analgesics and Antipyretics", Ullmann's Encyclopedia of Industrial Chemistry, Sixth Edition, Wiley-VCH Verlag GmbH, Germany 2000, pages 1-22 and H. Buschmann, T. Christoph, E. Friderichs, C. Maul, B. Sundermann, "Analgesics - From Chemistry and Pharmacology to Clinical Application", 2002, Part II, Wifey-VCH Verlag, Germany. The respective parts of said literature descriptions are incorporated by reference and form part of the present disclosure.

A specific embodiment of the present invention is a combination comprising
(a) (1R,2R)-3-(3-Dimethylamino-l-ethyl-2-methyl-propyl)-phenol, or the hydrochloride addition salt thereof, and (b) Paracetamol.

Both components (a) and (b) as part of the inventive combination may be administered in their usual daily dosage. The daily dosage of paracetamol should preferably not exceed 4g for adults. For infants and children the daily dosage should preferably not exceed 90 mg/kg. Preferably the compound (1R,2R)-3-(3-Dimethylamino-1-ethyl-2-methyl-propyl)-phenol may be administered to a patient in a daily dosage of 25 to 1000 mg, particularly preferably in a dosage of 50 to 800 mg, more particularly preferably in a dosage of 100 to 600 mg.

In another embodiment of the present invention the inventive combination may contain components (a) and (b) essentially in an equieffective ratio.

In yet a further embodiment of the inventive combination components (a) and (b) are present in such a weight ratio that the resulting composition will exert a synergistic effect upon administration to a patient. Suitable weight ratios can be determined by methods well known to those skilled in the art, e.g. via the Randall-Selitto test described below.

Both components (a) and (b) may also be present in the inventive combination in ratios deviating from the equieffective ratio. For, example, each of the components could be present in a range from 1/5 of the equieffective amount to 5 times the equieffective amount, preferably 1/4 to 4, more preferably 1/3 to 3, yet more preferably 1/2 to 2 of the equieffective amount.

In another embodiment of the present invention the components (a) and (b) can be administered in a specific dosage regimen to treat pain, for example, chronic pain or acute pain. Components (a) and (b) may be administered simultaneously or sequentially to one another, in each case via the same or different administration pathways. Another aspect of the present invention is therefore a method of treating pain, e.g. chronic or acute pain, **characterized in that** components (a) and (b) are administered simultaneously or sequentially to a mammal, wherein component (a) may be administered before or after component (b) and wherein components (a) or (b) are administered to the mammal either via the same or a different pathway of administration. Suitable pathways of administrations include but are not limited to oral, intravenous, intraperitoneal, transdermal, intrathekal, intramuscular, intranasal, transmucosal, subcutaneous, or rectal administration.

The inventive combinations are toxicologically safe and are therefore suitable for the treatment of mammals, particularly humans including infants, children and grownups.

Thus, in a further aspect the present invention relates to a pharmaceutical composition comprising an inventive combination as described herein and one or more auxiliary agents.

In a further aspect the present invention relates to a pharmaceutical dosage form comprising an inventive combination as described herein and one or more auxiliary agents.

In one embodiment the inventive pharmaceutical dosage form additionally comprises caffeine.

In one embodiment, the inventive pharmaceutical dosage form is suitable for being administered orally, intravenously, intraperitoneally, transdermally, intrathekally, intramuscularly, intranasally, transmucosally, subcutaneously, or rectally.

The inventive formulations and dosage forms may contain auxiliary agents, for example, carriers, fillers, solvents, diluents, colorants and/or binders. The selection of auxiliary agents and of the amounts of the same to be used depends, for example, on how the drug is to be administered, e.g. orally, intravenously, intraperitoneally, intradermally, intramuscularly, intranasally or locally, for example for infections of the skin, of the mucous membranes or of the eye.

Suitable auxiliary agents in the context of this invention are any substances known to a person skilled in the art useful for the preparation of galenical formulations. Examples of suitable auxiliary agents include but are not limited to: water, ethanol, 2-propanol, glycerol, ethylene glycol, propylene glycol, polyethylene glycol, polypropylene glycol, glucose, fructose, lactose, saccharose, dextrose, molasses, starch, modified starch, gelatine, sorbitol, inositol, mannitol, microcrystalline cellulose, methyl cellulose, carboxymethyl cellulose, cellulose acetate, shellac, cetyl alcohol, polyvinyl pyrrolidone, paraffins, waxes, natural and synthetic gums, acacia gum, alginates, dextran, saturated and unsaturated fatty acids, stearic acid, magnesium stearate, zinc stearate, glycerol stearate, sodium lauryl sulphate, edible oils, sesame oil, coconut oil, peanut oil, soybean oil, lecithin, sodium lactate, polyoxyethylene and polypropylene fatty acid ester, sorbitan fatty acid ester, sorbic acid, benzoic acid, citric acid, ascorbic acid, tannic acid, sodium chloride, potassium chloride, magnesium chloride, calcium chloride, magnesium oxide, zinc oxide, silicon dioxide, titanium oxide, titanium dioxide, magnesium sulphate, zinc sulphate, calcium sulphate, potash, calcium phosphate, dicalcium phosphate, potassium bromide, potassium iodide, talcum, kaolin, pectin, crosspovidone, agar and bentonite.

Pharmaceutical formulations (dosage forms) in the form of tablets, effervescent tablets, chewing tablets, dragees, capsules, drops, juices or syrups are, for example, suitable for oral administration. Oral pharmaceutical formulations may also be in the form of multiparticulates such as granules, pellets, spheres, crystals and the like, optionally compressed into a tablet, filled into a capsule, filled into a sachet or suspended in a suitable liquid medium. Oral pharmaceutical formulations may also be equipped with an enteric coating.

Pharmaceutical formulations that are suitable for parenteral, topical and inhalative administration include but are not limited to solutions, suspensions, easily reconstitutable dry preparations and sprays.

Suppositories are a suitable pharmaceutical formulation for rectal administration. Formulations in a deposit, in dissolved form, for example, in a patch optionally with the addition of agents to promote skin penetration, are examples of suitable formulations for percutaneous administration.

One or both of the components (a) and (b) may be present in the inventive pharmaceutical formulation at least partially in controlled-release form. Moreover, any controlled release/immediate release combination of said components may also be present in the inventive pharmaceutical formulation. For example, one or both of the components may be released from the inventive formulations with a certain delay, e.g. if administered orally, rectally or percutaneously. Such formulations are particularly useful for "once-daily" or "twice-daily" preparations, which only have to be taken once a day, respectively, twice a day. Suitable controlled-release materials are well known to those skilled in the art.

The inventive pharmaceutical formulations may be produced using materials, means, devices and processes that are well known in the prior art of pharmaceutical formulations, as described for example in "Remington's Pharmaceutical Sciences", A.R. Gennaro (ed.), 17th edition, Mack Publishing Company, Easton, Pa. (1985), in particular in part 8, chapters 76 to 93.

In order to obtain a solid pharmaceutical formulation such as a tablet, for example, the components of the pharmaceutical composition may be granulated with a pharmaceutical carrier, for example conventional tablet ingredients such as corn starch, lactose, saccharose, sorbitol, talcum, magnesium stearate, dicalcium phosphate or pharmaceutically acceptable gums, and pharmaceutical diluents, for example water, in order to form a solid composition that contains the components in homogeneous distribution. The term "homogeneous distribution" is taken to mean that the components are distributed uniformly over the entire composition, so that said composition may easily be divided into equally effective unit dose forms, such as tablets, pills or capsules. The solid composition is then divided into unit dose forms. The tablets or pills of the pharmaceutical composition according to the invention may also be coated or compounded in a different manner, in order to provide a dose form with a controlled release.

If one of the components, e.g. component (b), is to be released prior to the other component, for example at least 30 minutes or 1 hour beforehand, pharmaceutical formulations having a corresponding release profile may be prepared. An example of such a formulation is an osmotically driven release system for achieving a delayed release of component (a) via a coating that itself contains component (b) which is accordingly released earlier. In a release system of this kind, which is particularly suitable for oral administration, at least part, and preferably all, of the surface of the release system, preferably those parts that will come into contact with the release medium, is/are semipermeable, preferably equipped with a semipermeable coating, so the surface(s) is/are permeable to the release medium, but substantially, preferably entirely, impermeable to the active ingredient, component (a), the surface(s) and/or optionally the coating comprising at least one opening for releasing the active ingredient, component (a). Moreover, precisely that/those surface(s) that is/are in contact with the release medium is/are provided with a coating containing and releasing the other component, component (b). This is preferably taken to mean a system in tablet form comprising a release opening, an osmotic pharmaceutical composition core, a semipermeable membrane and a polymer portion that exerts pressure upon swelling. A suitable example of this kind of system is the system distributed by ALZA Corporation, USA under the tradenames OROS®, in particular, the OROS® Push-Pull^{™} System, the OROS® Delayed Push-Pull^{™} System, the OROS® Multi-Layer Push-Pull^{™} system, the OROS® Push-Stick System and also, in specific cases, the L-OROS^{™}.

Embodiments and examples of osmotically driven release systems are, for example, disclosed in US patents 4,765,989, 4,783,337 and 4,612,008.

A further example of a suitable pharmaceutical formulation is a gel-matrix tablet, such as the products developed by Penwest Pharmaceuticals (for example, under TimeRX). Suitable examples are provided in US patents 5,330,761, 5,399,362, 5,472,711 and 5,455,046, Particularly suitable is a retarding matrix formulation, with an inhomogeneous distribution of the pharmaceutically active composition, whereby, for example, the component (b) can be distributed in the outer region (the portion that comes into contact with the release medium most quickly) of the matrix and the other component (a) is distributed inside the matrix. On contact with the release medium, the outer matrix layer initially (and rapidly) swells and firstly releases the Paracetamol component, followed by the significantly (more) retarded release of component (a). Examples of a suitable matrix include matrices with 1 to 80 % by weight of one or more hydrophilic or hydrophobic polymers as pharmaceutically acceptable matrix formers. A further example of a suitable matrix may be inferred from US 4,389,393 the respective contents of which hereby being incorporated by reference and forming part of the disclosure of the present invention.

The amount of the inventive pharmaceutically active combination to be administered to the patient may vary depending on different factors well known to those skilled in the art, for example, the weight of the patient, the route of administration, or the severity of the illness.

In a further aspect the present invention relates to the use of an inventive combination as described herein for the preparation of a medicament for the treatment of pain.

In another embodiment the present invention relates to the use of an inventive combination as described herein for the preparation of a medicament for the treatment of pain, wherein the pain is selected from inflammatory pain, neuropathic pain, acute pain, chronic pain, visceral pain, migraine pain and cancer pain.

In yet another aspect the present invention relates to a method of treating pain in a mammal, preferably a human, which comprises administering an effective amount of an inventive combination as described herein to the mammal.

### Pharmacological methods:

### A. Randall-Selitto test in rats

The weight ratios of the components (a) and (b) that will lead to a supra-additive effect (synergistic effect) of the inventive pharmaceutical composition may be determined via the test of Randall and Selitto as described in Arch. Int. Pharmacodyn., 1957, 111: 409 to 419, which is a model for inflammatory pain. The respective part of the literature is hereby incorporated by reference and forms part of the present disclosure.

By means of injection of 0.1 ml of Carrageenin-suspension ventrally into a hind paw of a rat an oedema is induced, on which pain is generated 4 hours later by continuously increasing pressure with a stamp (2 mm tip diameter). The antinociceptive and antihyperalgesic activity of the tested substance is determined at different points in time after administration of the substance. The measured value to be determined and at the same time also the end point of the pain test is the pressure at which the vocalisation reaction of the rat occurs. The percentage maximum possible effect (%MPE) is calculated. The maximum pressure of the stamp is 250 g. The group size is n = 10.

The analysis of the results with respect to a supra-additive effect of the inventive pharmaceutical composition comprising the components (a) and (b) is carried out via statistical comparison of the theoretical additive ED₅₀-value with the experimentally determined ED₅₀-value of a so-called fixed ratio combination (isobolographic analysis according to Tallarida JT, Porreca F, and Cowan A. Statistical analysis of drug-drug and site-site interactions with isobolograms. Life Sci 1989; 45: 947 - 961).
The interactions studies presented herein were performed using equieffective doses of the two components, calculated from the ratio of the respective ED₅₀ values of the components if administered alone.

The application route was intravenous (i.v.) for (1R,2R)-3-(3-Dimethylamino-1-ethyl-2-methyl-propyl)-phenol (A) and intraperitoneal (i.p.) for Paracetamol. When A was applied alone, the peak effect was reached 15 min p. appl. (timepoint of first measurement) and an ED₅₀-value of 1.878 (1.694-2.065) mg/kg i.v. was calculated. Paracetamol induced a dose-dependent analgesic effect with an ED₅₀-value of 189,9 (181,3 -198,4) mg/kg i. p. respectively, reaching the peak effect 120 min p. appl. According to their respective timepoint of peak effect, (1R,2R)-3-(3-Dimethylamino-1-ethyl-2-methyl-propyl)-phenol was applied 15 min and Paracetamol 120 min before timepoint of measurement of the interaction-experiments (i. e. Paracetamol was applied 105 min before (1R,2R)-3-(3-Dimethylamino-1-ethyl-2-methyl-propyl)-phenol, respectively). Thus, the time point of ED₅₀ calculation of the combination corresponds to the timepoint of the peak effect of the respective compound. The isobolographic analysis revealed that the experimental ED₅₀-values of the combinations were significantly lower than the respective theoretical ED₅₀-values. Thus, the combination studies demonstrate significant synergistic interaction of (1R,2R)-3-(3-Dimethylamino-1-ethyl-2-methyl-propyl)-phenol with Paracetamol.

The results of the isobolographic analysis are summarized in the following table.

Experimental ED₅₀ values of A and Paracetamol and isobolographic analysis of the interaction between A and Paracetamol:

| | A | Paracetamol | Theoretical ED50 of the combination of A and Paracetamol | Experimental ED50 of the combination of A and Paracetamol | interaction |
|---|---|---|---|---|---|
| Substance / ED50 [mg/kg] (confidence interval) | 1,878 (1,694-2,065)* | 189,9 (181,3-198,4) | 95,90 (90,75 -101,0) | 74,88 (66,63 -84,17) | supra-additive (p < 0.001) |

| | | | | | |
|---|---|---|---|---|---|
| p: Level of statistical significance | | | | | |

From table 1 given above, the ratio of A to Paracetamol can be calculated to be 1:101

### Example:

### Preparation of a Paracetamol combination tablet

| Composition | |
|---|---|
| Paracetamol | 5000 g |
| (1R,2R)-3-(3-Dimethylamino-1-ethyl-2-methyl-propyl)-phenol | 500 g |
| Povidone K25 | 100 g |
| Microcrystalline Cellulose | 300 g |
| Powdered Cellulose | 140 g |
| Stearic Acid | 60 g |

Povidone is dissolved in 1.5 liter of water. Paracetamol and (1R,2R)-3-(3-Dimethylamino-1-ethyl-2-methyl-propyl)-phenol are blended in a high shear mixer and the compounds are granulated by adding the povidone solution. The wet mass is sized through a 3 mm sieve and dried in an oven at 50°C. The dry mass is sized together with microcrystalline cellulose and powdered cellulose through an 1 mm sieve. The mass is blended together with the stearic acid that has been passed through an 0.315 mm sieve. The final mass is pressed on an Korsch EKO tablet press into tablets of 13 mm diameter and a weight of 610 mg each.

## Claims

1. A combination comprising as component(s):
(a) at least one 3-(3-Dimethylamino-1-ethyl-2-methyl-propyl)-phenol of formula (I), 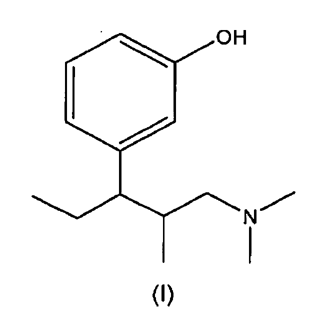 optionally in form of one of its pure stereoisomers, in particular an enantiomer or a diastereomer, a racemate or in form of a mixture of its stereoisomers, in particular enantiomers and/or diastereomers in any mixing ratio, or any corresponding acid addition salt thereof, or any solvate thereof, and
(b) Paracetamol.

2. Combination according to claim 1, **characterized in that** component (a) is selected from
(1R,2R)-3-(3-Dimethylamino-1-ethyl-2-methyl-propyl)-phenol,
(1S,2S)-3-(3-Dimethylamino-1-ethyl-2-methyl-propyl)-phenol,
(1R,2S)-3-(3-Dimethylamino-1-ethyl-2-methyl-propyl)-phenol,
(1S,2R)-3-(3-Dimethylamino-1-ethyl-2-methyl-propyl)-phenol, and any mixture thereof.

3. Combination according to claim 2, **characterized in that** component (a) is selected from
(1R,2R)-3-(3-Dimethylamino-1-ethyl-2-methyl-propyl)-phenol,
(1S,2S)-3-(3-Dimethylamino-1-ethyl-2-methyl-propyl)-phenol, and any mixture thereof.

4. Combination according to claim 2 or 3, **characterized in that** component (a) is the compound (1R,2R)-3-(3-Dimethylamino-1-ethyl-2-methyl-propyl)-phenol of formula (I'), 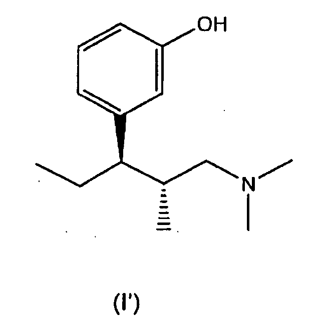 or an acid addition salt thereof, whereby the acid addition salt of hydrochloride is preferred.

5. Combination according to any of claims 1-4, **characterized in that** components (a) and (b) are present in such a weight ratio that the composition will exert a synergistic effect upon administration to a patient.

6. A pharmaceutical composition comprising a combination according to any one of claims 1-5 and optionally one or more auxiliary agents.

7. A dosage form comprising a combination according to any one of claims 1-5 and optionally one or more auxiliary agents.

8. A dosage form according to claim 7, **characterized in that** it is suitable for oral, intravenous, intraperitoneal, intradermal, intrathekal, intramuscular, intranasal, transmucosal, subcutaneous, or rectal administration.

9. A dosage form according to claim 7 or 8, **characterized in that** one or both of the components (a) and (b) is/are present in controlled-release form.

10. A dosage form according to any one of claims 7-9, **characterized in that** it additionally comprises caffeine.

11. Use of a combination according to any one of claims 1-5 for the preparation of a medicament for the treatment of pain.

12. Use according to claim 11, **characterised in that** the pain is selected from inflammatory pain, neuropathic pain, acute pain, chronic pain, visceral pain, migraine pain and cancer pain.

13. Use according to claim 11 or 12, **characterised in that** the pain is selected from inflammatory pain, neuropathic pain, acute pain, chronic pain, visceral pain, migraine pain and cancer pain.

14. Use according to any one of claims 11-13, **characterised in that** the medicament is adapted for simultaneous or sequential administration, wherein compound (a) may be administered before or after compound (b) and wherein compounds (a) or (b) are administered either by the same or a different pathway of administration.

## Patentansprüche

1. Kombination, die als Komponente(n) Folgendes umfasst:
(a) wenigstens ein 3-(3-Dimethylamino-1-ethyl-2-methyl-propyl)-phenol der Formel (I), 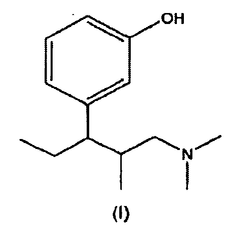 optional in Form eines seiner reinen Stereoisomere, insbesondere eines Enantiomers oder eines Diastereomers, eines Racemats oder in Form eines Gemischs seiner Stereoisomere, insbesondere Enantiomere und/oder Diastereomere in einem beliebigen Mischungsverhältnis, oder jedes beliebige entsprechende Säureadditionssalz davon oder jedes beliebige Solvat davon und
(b) Paracetamol.

2. Kombination nach Anspruch 1, **dadurch gekennzeichnet, dass** die Komponente (a) ausgewählt ist aus
(1R,2R)-3-(3-Dimethylamino-1-ethyl-2-methyl-propyl)-phenol,
(1S,2S)-3-(3-Dimethylamino-1-ethyl-2-methyl-propyl)-phenol,
(1R,2S)-3-(3-Dimethylamino-1-ethyl-2-methyl-propyl)-phenol,
(1S,2R)-3-(3-Dimethylamino-1-ethyl-2-methyl-propyl)-phenol und jedem beliebigen Gemisch davon.

3. Kombination nach Anspruch 2, **dadurch gekennzeichnet, dass** die Komponente (a) ausgewählt ist aus
(1R,2R)-3-(3-Dimethylamino-1-ethyl-2-methyl-propyl)-phenol,
(1S,2S)-3-(3-Dimethylamino-1-ethyl-2-methyl-propyl)-phenol und jedem beliebigen Gemisch davon.

4. Kombination nach Anspruch 2 oder 3, **dadurch gekennzeichnet, dass** die Komponente (a) die Verbindung (1R,2R)-3-(3-Dimethylamino-1-ethyl-2-methylpropyl)-phenol der Formel (I') 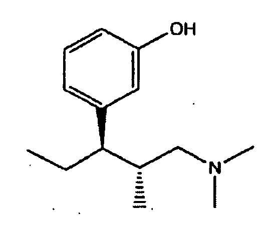 oder ein Säureadditionssalz davon ist, wobei das Säureadditionssalz von Hydrochlorid bevorzugt wird.

5. Kombination nach einem der Ansprüche 1-4, **dadurch gekennzeichnet, dass** die Komponenten (a) und (b) in einem solchen Gewichtsverhältnis vorliegen, dass die Zusammensetzung eine synergistische Wirkung nach der Verabreichung an einen Patienten ausüben wird.

6. Pharmazeutische Zusammensetzung, die eine Kombination nach einem der Ansprüche 1-5 und optional ein oder mehrere Hilfsmittel umfasst.

7. Dosierungsform, die eine Kombination nach einem der Ansprüche 1-5 und optional ein oder mehrere Hilfsmittel umfasst.

8. Dosierungsform nach Anspruch 7, **dadurch gekennzeichnet, dass** sie zur oralen, intravenösen, intraperitonealen, intradermalen, intrathekalen, intramuskulären, intranasalen, transmukosalen, subkutanen oder rektalen Verabreichung geeignet ist.

9. Dosierungsform nach Anspruch 7 oder 8, **dadurch gekennzeichnet, dass** eine oder beide der Komponenten (a) und (b) in kontrollierter Freisetzungsform vorliegt/vorliegen.

10. Dosierungsform nach einem der Ansprüche 7-9, **dadurch gekennzeichnet, dass** sie zusätzlich Koffein umfasst.

11. Verwendung einer Kombination nach einem der Ansprüche 1-5 zur Herstellung eines Medikaments zur Behandlung von Schmerzen.

12. Verwendung nach Anspruch 11, **dadurch gekennzeichnet, dass** der Schmerz ausgewählt ist aus Entzündungsschmerz, neuropathischem Schmerz, akutem Schmerz, chronischem Schmerz, viszeralem Schmerz, Migräneschmerz und Krebsschmerz.

13. Verwendung nach Anspruch 11 oder 12, **dadurch gekennzeichnet, dass** der Schmerz ausgewählt ist aus Entzündungsschmerz, neuropathischem Schmerz, akutem Schmerz, chronischem Schmerz, viszeralem Schmerz, Migräneschmerz und Krebsschmerz.

14. Verwendung nach einem der Ansprüche 11-13, **dadurch gekennzeichnet, dass** das Medikament für eine simultane oder sequenzielle Verabreichung vorgesehen ist, wobei die Verbindung (a) vor oder nach der Verbindung (b) verabreicht werden kann und wobei die Verbindungen (a) oder (b) entweder über dieselbe oder eine andere Verabreichungsroute verabreicht werden.

## Revendications

1. Combinaison comprenant en tant que composant(s) :
(a) au moins un 3-(3-diméthylamino-1-éthyl-2-méthyl-propyl)-phénol de formule (I), 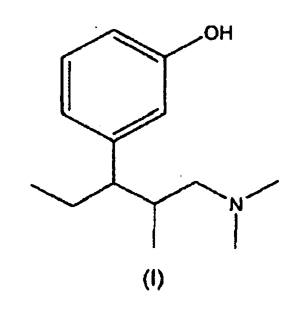 éventuellement sous forme de l'un de ses stéréoisomères purs, en particulier un énantiomère ou un diastéréoisomère, un racémate ou sous forme d'un mélange de ces stéréoisomères, en particulier des énantiomères et/ou diastéréoisomères dans un rapport quelconque, ou tout sel d'addition d'acides correspondant de celui-ci, ou tout solvate de celui-ci, et
(b)le paracétamol.

2. Combinaison selon la revendication 1, **caractérisée en ce que** le composant (a) est choisi parmi
le (1R,2R)-3-(3-diméthylamino-1-éthyl-2-méthylpropyl)-phénol,
le (1S,2S)-3-(3-diméthylamino-1-éthyl-2-méthylpropyl)-phénol,
le (1R,2S)-3-(3-diméthylamino-1-éthyl-2-méthylpropyl)-phénol
le (1S,2R)-3-(3-diméthylamino-1-éthyl-2-méthylpropyl)-phénol, et tout mélange de ceux-ci.

3. Combinaison selon la revendication 2, **caractérisée en ce que** le composant (a) est choisi parmi
le (1R,2R)-3-(3-diméthylamino-1-éthyl-2-méthylpropyl)-phénol,
le (1S,2S)-3-(3-diméthylamino-1-éthyl-2-méthylpropyl)-phénol, et tout mélange de ceux-ci.

4. Combinaison selon la revendication 2 ou 3, **caractérisée en ce que** le composant (a) est le composé (1R,2R)-3-(3-diméthylamino-1-éthyl-2-méthyl-propyl)-phénol de formule (I') 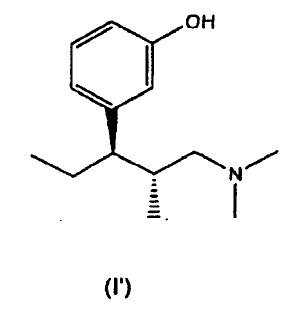 ou un sel d'addition d'acides de celui-ci, le sel d'addition d'acides de chlorhydrate étant préféré.

5. Combinaison selon l'une quelconque des revendications 1-4, **caractérisée en ce que** les composants (a) et (b) sont présents dans un rapport en poids tel que la composition exercera un effet synergique après administration à un patient.

6. Composition pharmaceutique comprenant une combinaison selon l'une quelconque des revendications 1-5 et éventuellement un ou plusieurs agents auxiliaires.

7. Forme de dosage comprenant une combinaison selon l'une quelconque des revendications 1-5 et éventuellement un ou plusieurs agents auxiliaires.

8. Forme de dosage selon la revendication 7, **caractérisée en ce qu'**elle est convenable pour l'administration par voie intraveineuse, intrapéritonéale, intradermique, intrathécale, intramusculaire, intranasale, transmuqueuse, sous-cutanée, ou rectale.

9. Forme de dosage selon la revendication 7 ou 8, **caractérisée en ce que** l'un des ou les deux composants (a) et (b) est/sont présent(s) sous une forme à libération contrôlée.

10. Forme de dosage selon l'une quelconque des revendications 7-9, **caractérisée en ce qu'**elle comprend en outre la caféine.

11. Utilisation d'une combinaison selon l'une quelconque des revendications 1-5 pour la préparation d'un médicament destiné au traitement de la douleur.

12. Utilisation selon la revendication 11, **caractérisée en ce que** la douleur est choisie parmi la douleur inflammatoire, la douleur neuropathique, la douleur aiguë, la douleur chronique, la douleur viscérale, la douleur migraineuse et la douleur cancéreuse.

13. Utilisation selon la revendication 11 ou 12, **caractérisée en ce que** la douleur est choisie parmi la douleur inflammatoire, la douleur neuropathique, la douleur aiguë, la douleur chronique, la douleur viscérale, la douleur migraineuse et la douleur cancéreuse.

14. Utilisation selon l'une quelconque des revendications 11-13, **caractérisée en ce que** le médicament est adapté à l'administration simultanée ou séquentielle, le composé (a) pouvant être administré avant ou après le composé (b) et les composés (a) ou (b) étant administrés soit par la même voie d'administration soit par des voies d'administration différentes.
